# NEW EUROPEAN PATENT SPECIFICATION

(11) **EP 0 487 105 B2**
(45) Date of publication and mention of the opposition decision: **11.12.2002**
(45) Mention of the grant of the patent: 27.03.1996
(21) Application number: 91119968.5
(22) Date of filing: 22.11.1991
(51) Int. Cl.: A61K 7/06, A61K 7/09

(54) **Waving lotion and method for use thereof**
Dauerwellenbildungslotion sowie Verfahren zur ihren Verwendung
Lotion pour onduler les cheveux et méthode pour son utilisation

(30) Priority: 22.11.1990 JP 32012690
(43) Date of publication of application: 27.05.1992
(73) Proprietor: YANAGITA COMPANY, Himeji-shi, Hyogo (JP)
(72) Inventor: Koyama, Takahiro, Hoya-shi, Tokyo (JP); Yanagita, Masahiro, Himeji-shi, Hyogo (JP)
(74) Representative: Grünecker, Kinkeldey, Stockmair & Schwanhäusser Anwaltssozietät

(56) References cited:
- EP-A- 0 085 894
- EP-A- 0 377 836
- CH-A- 307 483
- DE-A- 3 618 497
- DE-A- 3 833 681
- GB-A- 2 116 218
- GB-A- 2 185 269
- US-A- 4 299 817
- ZA-A- 7 201 483
- PATENT ABSTRACTS OF JAPAN, vol. 10, no. 362 (C-389)[2419], 4th December 1986; & JP-A-61 158 911 (DAICEL CHEM. IND. LTD) 18-07-1986
- SCHRADER, Karlheinz, Grundlagen und Rezepturen der Kosmetika, 2. Auflage. Hüthig Buch Verlag Heidelberg 1989, S. 840 Kap. 2.2.20
- Hugo Janistyn, Taschenbuch der modernen Parfümerie und Kosmetik, 4. Auflage, Wissenschaftliche Verlagsgemeinschaft m.b.H., Stuttgart, 1974, Kap. 7, S. 429,430
- Charles Zviak, The Science of Hair care, Marcel Dekker, Inc. New York, 1989, chapter 5, S. 208 2. und 3. Absatz

## Description

The present invention relates to a process for straightening curly hair comprising the use of a waving lotion and to the use of the waving lotion.

Although the mechanism of the permanent waving has not yet been fully elucidated, the cystine bonds (-S-S-) of the hair play an important role. Usually the first lotion (so-called waving lotion) and the second lotion are used for the permanent waving. The first lotion contains thioglycolic acid or its salt or cysteine as a reducing agent in the form of an alkaline solution. Water, the alkali and the reducing agent in the first lotion cut the hydrogen bond, salt bond and cystine bond, respectively, of the hair to soften it. Four oxidizing agents, i.e. potassium bromate, sodium bromate, sodium perborate and aqueous hydrogen peroxide solution, are usually admitted for the second lotion. Among them, sodium bromate is usually used. After the application of the first lotion, the hair is rinsed with an acidic rinse and the second lotion is applied thereto to regenerate the salt bond by the neutralization of the alkali, to regenerate the cystine bond by the effect of the oxidizing agent and to complete a new hydrogen bond as the hair is dried. Thus the intended waved hair can be obtained.

As for the function and quality required of the first lotion, the reducing power is indispensable and various concomitant conditions are also necessitated. Among them, a thickening spreader exerts a quite important influence thereon. The main purpose of the addition of the thickening spreader is to impart suitable viscosity and spreadability to the first lotion.

Ordinary thickening spreaders include cellulose derivatives, polysodium acrylate, polyisobutylene. polyethylene glycol. polyvinyl alcohol, natural pastes, etc. JP-A-286312/1986 discloses the use of a carboxymethylcellulose salt having a degree of etherification (hereinafter referred to as "DS") of at least 2.0 as the thickening spreader. However, waving lotions are used in various manners and the necessitated viscosity, function and properties vary depending on the use and manner of use thereof. Therefore, originality and idea are necessitated in using the thickening spreader depending on the use and manner of use thereof. JP-A-286312/1986 discloses only the use of the carboxymethylcellulose salt having a degree of etherification (hereinafter referred to as "DS") of at least 2 as the thickening spreader.

DE-A-3618497 discloses a composition for a waving lotion comprising an alkali metal salt or an ammonium salt of carboxymethylcellulose having a degree of etherification of 2.0 to 2.95 and a viscosity of 10 to 10,000 mPa.s in a 1% aqueous solution thereof at 20°C.

From GB-A-2116218 a permanent waving medium containing carboxymethylcellulose and having a viscosity of 5 to 100 mPa.s is known.

Various functions, properties and qualities are required of the thickening spreader of the waving lotion. Although the seriousness of the problems varies depending on the use and method of use thereof, the problems of the waving lotion include the stability of the function and quality over a long period of time, reducing power, suitable viscosity, preferred fluidity, uniform spreadability, affinity for the hair, high transparency, dispersion stability improving effect, possibility of curling at low temperature, rinsing effect, etc. Easiness of dissolution of the thickening spreader in the course of the production of the waving lotion is also important.

As for the use of the respective waving lotions, they include those for cold waving and those for permanent waving by ironing. A low viscosity, smooth fluidity, spreadability over the hair and easiness of intermediate washing with water and washing with an acid rinse are particularly required of the former, since the hair is reduced and oxidized in a state wound round a rod. A suitable viscosity and fluidity, affinity for the hair and curling effect at low temperature are particularly required of the latter, since the hair is thoroughly and uniformly reduced therewith and then curled by ironing. Further as for the waving lotions for straightening curly hair, a high viscosity and adhesion sufficient for forming the bundle of the hairs to prevent curling and thereby to straighten the hair in the step of applying the first lotion are required of them, since the curly hair must be straightened by reduction. In addition, it is required of them that they can be uniformly spread in an amount larger than that of the above-described other two kinds of the lotions.

Waving lotions for cold waving, lotions for waving by ironing and curly hair straightening lotions satisfying all of the above-described requirements can be obtained by using an alkali metal salt or ammonium salt of carboxymethylcellulose having a DS of at least 2.0 and controlling the viscosity in a specified range during the use.

It is the object underlying the present invention to provide a new process for straightening curly hair comprising the use of a waving lotion which can be uniformly spread on the hair and which forms hair bundles to straighten the hair to prevent curling thereof.

According to the present invention this object is attained with a process for straightening curty hair, characterized in that a waving lotion having a viscosity in the range of 7 to 60 Pa·s (7,000 to 60,000 cps) at a liquid temperature of 25° C, having a viscosity in the range of 3.2 to 39 Pa·s (3,200 to 39,000 cps) at a liquid temperature of 50°C and containing, as a thickening spreader, an alkali metal salt or ammonium salt of carboxymethylcellulose having a degree of etherification of at least 2.0, is applied to the hair for 8 to 15 min and the hair is left to stand for a processing time of 10 to 15 min without any pretreatment for straightening the curly hair.

The present invention also relates to the use of a waving lotion having a viscosity in the range of 7 to 60 Pa·s (7,000 to 60,000 cps) at a liquid temperature of 25°C, having a viscosity in the range of 3.2 to 39 Pa·s (3,200 to 39.000 cps) at a liquid temperature of 50°C and containing, as a thickening spreader, an alkali metal salt or ammonium salt of carboxymethylcellulose having a degree of ethertfication of at least 2.0 for straightening curly hair.

The waving lotion for straightening curly hair used according to the invention is characterized by containing, as a thickening spreader, an alkali metal salt or ammonium salt of carboxymethylcellulose having a DS of at least 2.0, the viscosity of the lotion during the use being in the range of 7 to 60 Pa.s (7,000 to 60,000 cps) at a liquid temperature of 25°C. and in the range of 3.2 to 39 Pa·s (3,200 to 39,000 cps) at a liquid temperature of 50°C.

The waving lotion used in the present invention comprises effective amounts of conventional ingredients which may include a reducing agent such as ammonium thioglycolate, sodium thioglycolate and cystein, an alkali such as sodium hydroxide and aqueous ammonium, a wetting agent such as propylene glycol, glycerin, cetyl alcohol and lanolin, a thickener such as carboxymethylcellulose and other additives such as an impregnating agent, a tonic, a colorant, an emulsifier, a perfume and a chelating agent and water, said carboxymethylcellulose being defined above.

It is preferable that the waving lotion composition comprises 1.0 to 7.5 % of the reducing agent. 0.15 to 0.57 % of an alkali. 3.0 to 8.0 % of the wetting agent. 1.0 to 15.0 % of the thickener, small amounts of other additives and 78 to 87 % of water, said carboxymethylcellulose being defined above, contained in an amount of 1.0 to 15.0 %. Percent here is based on weight.

It is important in using the waving lotion for straightening curly hair that a large amount thereof can be uniformly spread on the hair. An ideal lotion is one capable of forming hair bundles to straighten the hair and to prevent curling thereof in the step of applying the first lotion. A waving lotion for straightening curly hair satisfying the above-described conditions can be obtained by controlling the viscosity of the lotion as will be described below by using an alkali metal salt or ammonium salt of carboxymethylcellulose having a DS of at least 2.0 as the thickening spreader. The viscosity is 7 to 60 Pa.s (7.000 to 60.900 cps) when the liquid temperature is 25°C and 3.2 to 39 Pa.s (3.200 to 39.000 cps) when the liquid temperature is 50°C. When the waving lotion for straightening curly hair is used, no pretreatment for straightening curly hair is necessary and a waving lotion application time of 8 to 15 min and a processing time of 10 to 15 min are necessitated. Another process for straightening curly hair comprises applying the waving lotion to the hair, leaving the hair to stand for a processing time of 8 to 15 min and straightening the curly hair by ironing at 80 to 170°C.

The alkali metal salts of carboxymethylcellulose to be used in the waving lotion include lithium, potassium and sodium salts thereof, among which the sodium salt produced on an industrial scale is most usually used.

Ordinary components of waving lotions other than the thickening spreader can be suitably used depending on the use thereof.

### [Examples]

The following Examples will further illustrate the present invention.

### Examples 1 and 2

Earnest Gum FDM (trade name of ultrahigh-OS sodium salt of carboxymethylcellulose) as the thickening spreader in an amount specified in Table 1 was added to a commercially available waving lotion as a stock solution for cold waving to give waving lotions for various uses.
- Stock solution:: Kadas Beauty Wave N/F two-bath cold waving lotion for bristly hair
- manutacturer:: Wella Japan Co., Ltd.
- distributor:: Kadas Cosmetics Co., Ltd.

**[Table 1]**

| | | Earnest Gum FDM | | Use | Viscosity (cps) |
|---|---|---|---|---|---|
| | | DS | amount (g) | | |
| Ex. No. | 1 | 2.30 | 6.25 | straightening of curly hair | 8520 |
| | 2 | 2.10 | 11.25 | straightening of curly hair | 52000 |
| Stock solution | | | 0 | cold waving | 2.3 |

The amount specrfied in the Table indicates that of Earnest Gum FDM added to 100 g of the stock solution. The viscosity was determined with a Brookfield viscometer at 25°C.

The practical value of each of the waving lotions listed in the Table 1 used in various ways was evaluated as will be described below:

### Evaluation of straightening of curly hair:

A male beautician A (age: 40) and a female beautician B (age: 35) were each asked to straighten the curly hairs of 10 subjects with each of the waving lotions prepared in the Examples 1 and 2.

The results of the evaluation were summarized as follows:
(1) although the waving lotion had a high viscosity, it had a suitable fluidity and affinity for the hair and, therefore, it could be uniformly spread in a large amount without forming lumps unlike the ordinary waving lotions,
(2) since hair bundles were formed to accelerate the straightening when the first lotion was applied, the conditions in the subsequent step were made milder,
(3) after the completion of the straightening, the straightened hair was observed to find that it scarcely curled again with time, and (4) an excellent storability similar to that described above was recognized.

On the contrary, when the stock solution was used, the absolute amount of the spread solution was insufficient for the completion of the reduction and the object could not be attained, since the viscosity of the solution was insufficient in all the cases.

### Example 3 (Evaluation of straightening of curly hair: straightening method)

A male beautician A (age: 40) and a female beautician B (age: 35) were each asked to straighten the curly hairs of 10 subjects with each of the waving lotions for straightening curly hair prepared in the Examples 1 and 2 by the following straightening method and the results were evaluated.

### [Method (conditions)]

I. Precare:
   (1) diagnosis of hair: by an ordinary method
   (2) preshampooing: ditto
II. Selection of hair style: ditto
III. Application of reducing agent (the first lotion):
   (1) About 80 to 150 ml of the first lotion in gel form was applied to the hair in the range of 5 to 10 mm distant from the scalp to the tip of the hair by thoroughly combing the hair crosswise and lengthwise with a rough-toothed jumbo comb suitably for the volume of the hair for 8 to 15 min and then the hair was covered with a cap (processing time for the first lotion: 10 to 15 min).
   (2) After the processing, the hair was washed with slightly warm water to remove the first lotion.
   (3) The hair was dried with a towel to remove water from the root to the tip of the hair. The use of a hair dryer was not allowed at all.
IV. Application of oxidizing agent (the second lotion): by an ordinary method for straightening curly hair
V Rinsing with acid rinse (treatment): by an ordinary method
VI. Aftercutting:
   The hair was cut after fixing the length and volume of the hair according to the subject's request and taking the volume and direction of stream of the hair into consideration. Then the hair was further cut by suitably setting the volume, direction of stream and curling of the hair in order to dress the hair style as a whole.
VII. Application of hair restoring agent: by an ordinary method
VIII. Blowing and setting of the hair: by an ordinary method

### [Evaluation results]

(1) Although the waving lotion had a high viscosity, it had a suitable fluidity and affinity for the hair and, therefore, could be evenly spread in a large amount without forming lumps unlike the ordinary waving lotions.
(2) Since the waving lotion had suitable adhesion and affinity for the hair, it did not flow down to the scalp, and, therefore, did not damage the scalp.
(3) Since hair bundles were formed to accelerate the straightening when the first lotion was applied, the conditions in the subsequent step were made milder.
(4) Since the waving lotion having a stable viscosity could be applied to the hair, the operation time could be shortened by 20 to 90 min as compared with that of the conventional straightening method and the reduction time was shortened by about 20%.
(5) After the completion of the straightening, the straightened hair was observed to find that it scarcely curled again with time.
(6) After the completion of the straightening, the hair could be quite easily combed and a rinsing effect was recognized.

### Illustration Example 1

A male beautician A (age: 40) and a female beautician B (age: 35) were each asked to straighten the curly hairs of 10 subjects with a commercially available lotion for straightening curly hair by the following method and the results were evaluated.

### [Method (conditions)]

The method and conditions were the same as those of the Example 3.

### [Evaluation results]

(1) The straightening of curly hair was unsatisfactory.
(2) Since the operation time was as long as 150 to 200 min, the productivity was too low.
(3) The results of the straightening were variable depending on the worker.

### Comparative Example 1

A male beautician A (age: 40) and a female beautician B (age: 35) were each asked to straighten the curly hairs of 10 subjects with a commercially available waving lotion for straightening curly hair by the following straightening method and the results were evaluated.

### [Method (conditions)]

I. Precare:
   (1) diagnosis of hair: by an ordinary method
   (2) preshampooing: ditto
II. Selection of hair style: ditto
III. Application of reducing agent (the first lotion):
   (1) The hair was pretreated by applying a treatment or the first lotion or by treating it so that it would be easily reduced. The hair was heated for 10 to 20 min or it was left to stand for a processing time of 10 to 20 min.
   (2) About 80 to 150 ml (suitably determined depending on the volume of the hair) of the first lotion in creamy form was evenly applied to the hair (from the root to the tip) by thoroughly combing the hair crosswise and lengthwise with a rough-toothed jumbo comb in such a manner that it would not adhere to the scalp and then the hair was covered with a cap (processing time for the first lotion: 10 to 15 min under heating and then 10 to 15 min at ambient temperature).
   (3) After the processing, the hair was washed with slightly warm water to remove the first lotion.
   (4) The hair was dried well with a towel to remove water from the root to the tip of the hair. The use of a hair dryer was not allowed at all.
IV Application of oxidizing agent (the second lotion):
   The second lotion in creamy form was applied to the reduced hair and the straightened hair was left to stand for a processing time of 10 to 20 min.
V. Rinsing with acid rinse (treatment): by an ordinary method
VI. Aftercutting:
   The hair was out after fixing the length and volume of the hair according to the subject's request and taking the volume and direction of stream of the hair into consideration. Then the hair was further cut by suitably setting the volume, direction of stream and curling of the hair in order to dress the hair style as a whole.

### [Evaluation results]

(1) The hair became curly again in a considerable number of the subjects.
(2) The hair became frizzy.
(3) Since an operation time of 2 to 3.5 h was taken, the productivity was not improved. The subjects got tired.

### Example 4 (Evaluation of straightening of curly hair: iron straightening method)

A male beautician A (age: 40) and a female beautician B (age: 35) were each asked to straighten the curly hairs of 10 subjects with each of the waving lotions prepared in the Examples 1 and 2 by the following iron straightening method and the results were evaluated.

### [Method (conditions)]

I. Precare:
   (1) diagnosis of hair: by an ordinary method
   (2) preshampooing: ditto
II. Selection of hair style: ditto
III. Drying:
   The hair was dried to such an extent that it was still a little moist so as to facilitate the application of the first lotion.
IV Application of reducing agent (the first lotion):
   (1) About 50 to 100 ml/subject of the first lotion was applied to the hair (processing time for the first lotion: 8 to 15 min).
   (2) After the processing, the hair was washed with slightly warm water to remove the first lotion.
   (3) The hair was dried well with a towel to remove water from the root to the tip of the hair.
   (4) Application of treatment oil:

   A treatment oil was uniformly applied to the hair so that the ironing would be smooth and easy.
V Ironing:
   The hair was rapidly ironed in 10 to 30 min according to the selected hair style by heating to such a temperature that the surly hair could be straightened, which was as low as possible (130 to 160°C). The heating to a higher temperature or for a long period of time was to be avoided.
VI. Application of oxidizing agent (the second lotion): by an ordinary method for straightening curly hair
VII. Rinsing with acid rinse (treatment): by an ordinary method
VIII. Aftercutting:
   (1) The hair was cut after fixing the length and volume of the hair according to the subject's request and taking the volume and direction of stream of the hair into consideration.
   (2) Then the hair was further cut while observing the curling state of the hair in order to dress the hair style as a whole.
IX. Application of hair restoring agent: by an ordinary method

### [Evaluation results]

(1) Since the waving lotion had suitable adhesion and affinity for the hair, it did not flow down to the scalp and, therefore, did not damage the scalp.
(2) Although the waving lotion had a high viscosity, it had a suitable fluidity and affinity for the hair and, therefore, it could be uniformly spread in a large amount without forming lumps unlike the ordinary waving lotions.
(3) Since hair bundles were formed to accelerate the straightening when the first lotion was applied, the conditions in the subsequent step were made milder.
(4) After the completion of the operation, the straightened hair was observed to find that it scarcely curled again with time.
(5) Since the waving lotion having a stable viscosity was applied to the hair and the hair was straightened by ironing, the operation time could be shortened by 20 to 90 min as compared with that of the conventional straightening method and the reduction time was shortened by about 20%.
(6) After washing to remove the reducing agent, the hair was straightened with an iron. The curly hair could be surely straightened according to the hair design with the iron and the straightened hair was beautiful.
(7) Since the hair could be straightened by ironing at a temperature of as low as 80 to 170°C, the hair was not damaged.
(8) After the completion of the straightening, the hair could be quite easily combed and a rinsing effect was recognized.

### Illustration Example 2

A male beautician A (age: 40) and a female beautician B (age: 35) were each asked to straighten the curly hairs of 10 subjects with a commercially available waving lotion for straightening curly hair by the following iron straightening method and the results were evaluated.

### (Method (conditions)]

The method and conditions were the same as those of the Example 4.

### [Evaluation results]

(1) The hair was occasionally damaged or broken.
(2) Water contained in the hair began to vaporize in the course of ironing.
(3) Even when the hair was straightened with an iron while the hair was dried, the hair was curled again due to water contained in the oxidizing agent.
(4) The hair was damaged and became frizzy.
(5) The straightness of the hair and the style thereof (including the volume and stream of the hair) desired by the subject could not be realized.

The following effects are obtained with the waving lotion for use in the present invention:
(1) the quality of the waving lotion can be kept over a long period of time.
(2) the availability of the first lotion is improved.
(3) the operation time can be shortened, so that it is possible to improve the productivity and the availability of the worker.
(4) The treatment temperature is lowered, so that the hair can be protected from thermal damage.
(5) Rinsing effect can be obtained and smooth combing becomes possible.

The process wherein the waving lotion of the present invention is used is far superior to the conventional processes.

### Straightening of curly hair:

The complicated process necessitating a long time can be simplified and only an operation according to the principle of cold waving is required. Therefore, the object, the straightening of curly hair, can be attained by the simple operation in a short period of time.

## Claims

1. Use of a waving lotion having a viscosity in the range of 7 to 60 Pa·s (7,000 to 60,000 cps) at a liquid temperature of 25°C, having a viscosity in the range of 3.2 to 39 Pa.s (3,200 to 39,000 cps) at a liquid temperature of 50°C and containing, as a thickening spreader, an alkali metal salt or ammonium salt of carboxymethylcellulose having a degree of etherification of at least 2.0 for straightening curly hair.

2. A process for straightening curly hair, **characterized in that** a waving lotion having a viscosity in the range of 7 to 60 Pa·s (7,000 to 60,000 cps) at a liquid temperature of 25° C, having a viscosity in the range of 3.2 to 39 Pa·s (3,200 to 39,000 cps) at a liquid temperature of 50°C and containing, as a thickening spreader, an alkali metal salt or ammonium salt of carboxymethylcellulose having a degree of etherification of at least 2.0, is applied to the hair for 8 to 15 min and the hair is left to stand for a processing time of 10 to 15 min without any pretreatment for straightening the curly hair.

3. A process for straightening curly hair, **characterized in that** the waving lotion set forth in claim 2 is applied to the hair and the hair is left to stand for a processing time of 8 to 15 min and then straightened by ironing.

## Patentansprüche

1. Verwendung einer Wellotion mit einer Viskosität im Bereich von 7 bis 60 Pa.s (7000 bis 60000 cps) bei einer Flüssigkeitstemperatur von 25 °C, mit einer Viskosität im Bereich von 3,2 bis 39 Pa.s (3200 bis 39000 cps) bei einer Flüssigkeitstemperatur von 50 °C, welche als Verdickungsmittel-Verteiler ein Alkalimetallsalz oder ein Ammoniumsalz von Carboxymethylcellulose mit einem Veretherungsgrad von mindestens 2,0 enthält, zur Glättung von lockigen Haaren.

2. Verfahren zur Glättung von lockigen Haaren, **dadurch gekennzeichnet, daß** eine Wellotion mit einer Viskosität im Bereich von 7 bis 60 Pa.s (7000 bis 60000 cps) bei einer Flüssigkeitstemperatur von 25 °C, mit einer Viskosität im Bereich von 3,2 bis 39 Pa.s (3200 bis 39000 cps) bei einer Flüssigkeitstemperatur von 50 °C, welche als Verdickungsmittel-Verteiler ein Alkalimetallsalz oder Ammoniumsalz von Carboxymethylcellulose mit einem Veretherungsgrad von mindestens 2,0 enthält, für 8 bis 15 Minuten auf das Haar aufgebracht wird und die Haare ohne jede Vorbehandlung zur Glättung der lockigen Haare für eine Einwirkungszeit von 10 bis 15 Minuten sich selbst überlassen bleiben.

3. Verfahren zur Glättung von lockigen Haaren, **dadurch gekennzeichnet, daß** die in Anspruch 2 erläuterte Wellotion auf das Haar aufgebracht wird und die Haare für eine Einwirkungszeit von 8 bis 15 Minuten sich selbst überlassen bleiben und dann durch Ondulieren geglättet werden.

## Revendications

1. Utilisation d'une lotion pour onduler les cheveux ayant une viscosité allant de 7 à 60 Pa.s (de 7000 à 60 000 cps) à une température du liquide de 25°C, ayant une viscosité allant de 3,2 à 39 Pa.s (de 3200 à 39 000 Cps) à une température du liquide de 50°C et contenant, comme agent de diffusion épaississant, un sel de métal alcalin ou un sel d'ammonium de carboxyméthylcellulose ayant un degré d'éthérification d'au moins 2.0 pour raidir des cheveux frisés.

2. Procédé pour raidir des cheveux frisés, **caractérisé en ce qu'**on applique une lotion pour onduler les cheveux ayant une viscosité allant de 7 à 60 Pa.s (de 7000 à 60 000 cps) à une température du liquide de 25°C, ayant une viscosité allant de 3,2 à 39 Pa.s (de 3200 à 39 000 cps) à une température du liquide de 50°C et contenant, en tant qu'agent de diffusion épaississant, un sel de métal alcalin ou un sel d'ammonium de carboxyméthylcellulose ayant un degré d'éthérification d'au moins 2.0, sur les cheveux pendant 8 à 15 minutes et on laisse reposer les cheveux pendant une durée de traitement de 10 à 15 minutes sans aucun pré-traitement pour raidir les cheveux frisés.

3. Procédé pour raidir des cheveux frisés, **caractérisé en ce qu'**on applique une lotion pour onduler les cheveux sus-mentionnée dans la revendication 2 sur les cheveux et on laisse reposer les cheveux pendant une durée de traitement de 8 à 15 minutes, puis on les raidit au fer à friser.
